(19) Europäisches Patentamt
European Patent Office
Office européen des brevets

(11) **EP 3 727 449 B1**

(12) **EUROPEAN PATENT SPECIFICATION**

(45) Date of publication and mention
of the grant of the patent:
**05.03.2025 Bulletin 2025/10**

(21) Application number: **18825727.3**

(22) Date of filing: **21.12.2018**

(51) International Patent Classification (IPC):
*A61K 31/351* (2006.01)    *A61K 45/06* (2006.01)
*A61K 9/10* (2006.01)    *A61K 31/496* (2006.01)
*A61K 31/575* (2006.01)    *A61K 31/7036* (2006.01)
*A61K 33/38* (2006.01)    *A61P 31/04* (2006.01)
*A61K 47/02* (2006.01)

(52) Cooperative Patent Classification (CPC):
(C-Sets available)
**A61K 45/06; A01N 25/08; A61K 9/10;**
**A61K 31/351; A61K 31/496; A61K 31/575;**
**A61K 31/7036; A61K 33/38; A61K 47/02;**
**Y02A 50/30**                    (Cont.)

(86) International application number:
**PCT/EP2018/086790**

(87) International publication number:
**WO 2019/122429 (27.06.2019 Gazette 2019/26)**

(54) **PHARMACEUTICAL COMPOSITION COMPRISING A COLLOIDAL DISPERSION AND A THERAPEUTIC AGENT AND METHODS AND USES THEREOF**

PHARMAZEUTISCHE ZUSAMMENSETZUNG MIT EINER KOLLOIDALEN DISPERSION UND EINEM THERAPEUTIKUM SOWIE VERFAHREN UND VERWENDUNGEN DAVON

COMPOSITION PHARMACEUTIQUE COMPRENANT UNE DISPERSION COLLOÏDALE ET UN AGENT THÉRAPEUTIQUE ET SES PROCÉDÉS ET SON UTILISATIONS

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **21.12.2017 EP 17209879**

(43) Date of publication of application:
**28.10.2020 Bulletin 2020/44**

(73) Proprietor: **PREBONA AB**
**272 36 Simrishamn (SE)**

(72) Inventors:
• **AHLNECK, Claes**
**431 35 Mölndal (SE)**
• **ANDERSSON, Dan**
**756 44 UPPSALA (SE)**

(74) Representative: **Brann AB**
**P.O. Box 3690**
**Sveavägen 63**
**103 59 Stockholm (SE)**

(56) References cited:
**WO-A1-2011/037523    CN-A- 105 998 062**

• **LU MENGMENG ET AL: "Synergistic bactericidal activity of chlorhexidine-loaded, silver-decorated mesoporous silica nanoparticles", INTERNATIONAL JOURNAL OF NANOMEDICINE, vol. Volume 12, 1 May 2017 (2017-05-01), pages 3577 - 3589, XP055972884, Retrieved from the Internet <URL:https://www. dovepress.com/getfile.php?fileID=36397> DOI: 10.2147/IJN.S133846**

- **JEONG-KI JO ET AL: "Rechargeable microbial anti-adhesive polymethyl methacrylate incorporating silver sulfadiazine-loaded mesoporous silica nanocarriers", DENTAL MATERIALS, vol. 33, no. 10, 1 October 2017 (2017-10-01), AMSTERDAM, NL, pages e361 - e372, XP055565217, ISSN: 0109-5641, DOI: 10.1016/j.dental.2017.07.009**
- **GUANGYIN LEI: "Synthesis of nano-silver colloids and their anti-microbial effects", 8 November 2007 (2007-11-08), XP055478547, Retrieved from the Internet <URL:https://pdfs.semanticscholar.org/3bef/f6d218aa569609c939e11806b8c88fbc5e35.pdf> [retrieved on 20180525]**
- **SHIVA K RASTOGI ET AL: "Ag colloids and Ag clusters over EDAPTMS-coated silica nanoparticles: synthesis, characterization, and antibacterial activity against", NANOMEDICINE: NANOTECHNOLOGY, BIOLOGY AND MEDICINE, ELSEVIER, NL, vol. 7, no. 3, 3 November 2010 (2010-11-03), pages 305 - 314, XP028219878, ISSN: 1549-9634, [retrieved on 20101119], DOI: 10.1016/J.NANO.2010.11.003**
- **PAUL SAVAGE ET AL: "Antibacterial properties of cationic steroid antibiotics.", FEMS MICROBIOLOGY LETTERS, vol. 217, no. 1, 1 November 2002 (2002-11-01), pages 1 - 7, XP055068591, ISSN: 0378-1097, DOI: 10.1111/j.1574-6968.2002.tb11448.x**
- **POULIKAKOS P ET AL: "Aminoglycoside therapy in infectious diseases", EXPERT OPINION ON PHARMACOTHE, ASHLEY PUBLICATIONS LTD, LONDON, UK, vol. 14, no. 12, 1 August 2013 (2013-08-01), pages 1585 - 1597, XP009179074, ISSN: 1465-6566, DOI: 10.1517/14656566.2013.806486**
- **N. MESAROS ET AL: "Pseudomonas aeruginosa: resistance and therapeutic options at the turn of the new millennium", CLINICAL MICROBIOLOGY AND INFECTION., vol. 13, no. 6, 1 June 2007 (2007-06-01), United Kingdom, Switzerland, pages 560 - 578, XP055478541, ISSN: 1198-743X, DOI: 10.1111/j.1469-0691.2007.01681.x**

(52) Cooperative Patent Classification (CPC): (Cont.)

C-Sets
A01N 25/08, A01N 59/16;
A61K 31/351, A61K 2300/00;
A61K 31/496, A61K 2300/00;
A61K 31/575, A61K 2300/00;
A61K 31/7036, A61K 2300/00;
A61K 33/38, A61K 2300/00

# EP 3 727 449 B1

**Description**

TECHNICAL FIELD OF THE INVENTION

The present invention relates to the field of pharmaceutical compositions comprising colloidal dispersions of particles of silica to which particles silver ions have been adsorbed in combination with therapeutically active agents. It is also related to a kit of parts, uses and medical methods of treatment involving said pharmaceutical compositions.

BACKGROUND OF THE INVENTION

Nanotechnologies are the science and business of manipulating matter at the atomic scale. Materials produced with the aid of various kinds of nanotechnologies are starting to be used in many areas of everyday life such as medicine, cosmetics, clothing fabrics, sports equipment, paints, packaging, food, etc. and has been used for some time as for instance catalysts in many important industrial processes. In the future we will no doubt see many more application of nanomaterials in general and of nanomaterials involving noble metals in particular.

Transition metal ions and copper ions, either alone or in copper complexes, in particular, have been used to disinfect liquids, solids and human tissue for centuries. Today, copper is used as a water purifier, algaecide, fungicide, nematocide, molluscicide as well as an anti-bacterial and anti-fouling agent. Copper also displays potent anti-viral activity. Complexes of divalent transition metals like Ni(II), Co(II), Cu(II) and Zn(II), as well as lanthanide ions, with Schiff's base ligands have been shown to possess bactericidal and/or fungicidal activities.

Prior art describes the preparation and use of colloidal forms of transition metals, for instance colloidal silver and copper, as biocides in various applications. Particle size and particle size distribution are often described as important properties of such colloidal dispersions although their values are rarely specified. In some prior art it is stated that it is desirable that at least 50 % of the particles have a particle size smaller than 15 nanometers. When nanoparticles of noble metals are used as biocides, or to provide other functions, only the metal atoms at the surface of the particles are able to come into contact and interact with microbes or different kinds or with reactants to provide antimicrobial effect or e.g. to promote the formation of products. Metal atoms in the interior of the particles have no access to the environment outside of the particles and therefore have no biocidal - or other - activity. Let $n_s$ and $n_t$ denote the number of surface atoms and the total number of atoms, for instance noble metal atoms, respectively. The ratio $n_s/n_t$ is called the degree of dispersion of the noble metal and is a very important property of noble metals in application, for instance many catalytic applications or biocidal applications, where their performance depend on the number of atoms they expose to their environment. The degree of dispersion decreases rapidly with particle size. In the case of, for instance, nano-particles of silver, the degree of dispersion thus decreases from about 85 % to about 30 % when the particle size grows from 1 nanometer to 5 nanometers. The degree of dispersion of 15 nanometer particles is less than 10 %, indicating that more than 90 % of the metal resides inactive in the interior of the particles.

There is an effort to incorporate silver nanoparticles, which have an antimicrobial effect that lasts longer than ionic silver, into a wide range of medical devices, including but not limited to bone cement, surgical instruments, surgical masks, wound dressings.

WO 2008/079149 A1 describes an antimicrobial formulation for dental applications that includes colloidal silver, from between about 0.01 to 2 %, and colloidal copper, from between about 0.05 to 10 %.

WO 2008/024422 A2 discloses incorporation of colloidal silver in compositions for use in partially or fully decontaminating surfaces which have been contaminated with chemical or biological warfare agents as well as to methods for treating viral infections, bacterial infections, fungal infections, and cancerous tissue.

In WO 2008/147427 A2, a colorless composition comprising silver particles and water is disclosed. The particles have an interior of elemental silver and an exterior of ionic silver oxide, wherein the silver particles are present in the water at a level of about 5-40 ppm. A preferred embodiment of the invention is a silver composition comprising particles of silver wherein more than 50% of the particles are less than 0.015 micrometers in size and the particles are colloidally suspended in water. The composition manifests significant antiviral properties and is effective against avian influenza virus. Methods of use of the composition are described.

WO 2009/036714 A1 discloses a composition for the treatment of wounds, containing hyaluronic acid, urea, and colloidal silver.

WO2011037523 describes biocidal colloidal dispersions of silica particles with silver ions adsorbed thereon usable as biocides.

CN105998062 discloses silver-PVP or silver-citrate nanoparticles having a particle size of 20-30 nm to show synergistic anti-bacterial activity when combined with the aminoglycoside kanamycin.

Still, there is a constant need for improved and/or alternative pharmaceutical compositions possessing antibiotic activity, to reduce the extensive use of traditional antibiotics.

SUMMARY OF THE INVENTION

In a first aspect, the present invention relates to a pharmaceutical composition comprising an antibiotic agent as defined in the appended claims and a colloidal dispersion of particles of silica having a particle size from 3 nm to 25 nm to which particles silver ions have been adsorbed.

In another aspect, there is also provided herein a kit of parts comprising a pharmaceutical formulation including an antibiotic agent as defined in the appended claims, optionally in admixture with a pharmaceutically acceptable excipient and a colloidal dispersion comprising particles of silica having a particle size from 3 nm to 25 nm to which particles silver ions have been adsorbed.

In yet another aspect there is provided a colloidal dispersion comprising particles of silica having a particle size from 3 nm to 25 nm to which particles silver ions have been adsorbed, for use in a method for the treatment of a bacterial infection, by administration in combination with an antibiotic agent as defined in the appended claims.

In yet another aspect there is provided an antibiotic agent as defined in the appended claims for use in a method for the treatment of a bacterial infection, by administration in combination with a colloidal dispersion comprising particles of silica having a particle size from 3 nm to 25 nm to which particles silver ions have been adsorbed.

BRIEF DESCRIPTION OF THE FIGURES

Figure 1: Highly sensitive competition experiments of example 1, with antibiotic agent Streptomycin alone or in combination with AgSol (Ag nano) or AgNO$_3$ (Ag), respectively (Fig 1A and Fig 1B), on *Salmonella enterica (S. enterica) Typhimurium* LT2.

Figures 2A-2F: AgSol-antibiotic synergy screening assay on *S. enterica Typhimurium* LT2 (example 2). Fig 2A-2C: AgSol (1000-5-5, 300-5-5) and AgNO$_3$ in combination with Streptomycin, respectively; Fig 2D-F; AgSol (1000-5-5, 300-5-5) and AgNO$_3$ in combination with rifampicin; respectively.

Figure 3: AgSol-antibiotic synergy screening assay on *Pseudomonas aeruginosa (P. aeruginosa)* PA01 with the antibiotic agent gentamicin (GEN) in combination with AgSol (1000-5-5) and AgNO$_3$, respectively (example 4).

Figure 4: AgSol-antibiotic synergy screening assay on *Staphylococcus aureus (S.aureus)* USA300 JE2, with fusidic acid (FUS) in combination with AgSol (1000-5-5) (example 4).

Figure 5: AgSol-antibiotic synergy screening assay on S. *aureus* USA300 JE2, and Mupirocin (MUP) in combination with AgSol (1000-5-5) (example 4).

Figure 6: Content of AgSol was varied (content of AgSol MIC was varied to 0%, 10%, 25% and 50% of MIC of silver (Minimal Inhibitory Concentration) and Streptomycin was constant *(Escherichia coli (E.coli)* MG1655). (Example 5)

Figure 7: "Time Kill" test with *S.aureus* USA300 JE2 and Fusidic acid (FUS) or Mupirocin (MUP), in combination with AgSol (Figure 7A and Figure 7B, respectively).

Figure 8: *P. aeruginosa* (PAO1) with gentamicin (GEN) in combination with AgSol or AgNO$_3$, lower inoculum (Figure 8A) and higher inoculum (Figure 8B).

Figure 9: AgSol-antibiotic synergy screening assay on gentamicin-resistant *Pseudomonas aeruginosa (P. aeruginosa)* isolate DA60255 with the antibiotic agent gentamicin (GEN) in combination with AgSol (1000-5-5).

DETAILED DESCRIPTION OF THE INVENTION

*Colloidal Silica*

A convenient source of the silica particles of the present invention are commercial silica sols. Such sols are aqueous dispersions of silica particles and the particles are uniform spheres of silica, which have no internal surface area or detectable crystallinity. They are usually dispersed in an alkaline medium, which reacts with the silica surface to produce a negative charge. Because of the negative charge, the particles repel one another resulting in a stable product.

In some commercial products, the particle surface is modified with aluminosilicate ions so as to provide the surface with a fixed, pH-independent negative charge that will make the products more stable towards gelling than the sols from which they were prepared. Trivalent aluminium atoms have been substituted for part of the tetravalent silicon atoms in the surface of the particles, creating a fixed negative charge which is independent of pH. The surface coverage of Al is much less than that corresponding to a Langmuir monolayer.

There are also commercial silica sols in which the particles have a positive charge and are dispersed in an acidic solution. The negative surface charge has been reversed by adsorption of octahedral aluminium ions such as those present in basic aluminium chloride.

The solids content depends on the particle size and varies from less than 10 % per weight silica for the smallest particles, 3 nm, to about 50 % per weight silica for larger particles, > 20 nm. The surface of the particles in aqueous silica sols is covered with surface hydroxyl groups, silanol groups. The particle size of commercial silica sols is typically in the range of 3-100 nm. The specific surface area (e.g. by SEARS's titration or BET) is generally of 25-1000 $m^2$/g, such as 30-800 $m^2$/g, more preferably 100-600 $m^2$/g, even more preferably 200-600 $m^2$/g, and most preferably 200-550 $m^2$/g.

Stabilization of commercial silica sols is accomplished by adjusting the pH of the sol to between 8.0 and 10.0 by addition of alkali, usually a solution of sodium hydroxide. The sols also contain small amounts of other electrolytes such as sodium chloride and sodium sulfate. The stability of highly concentrated silica sols is very sensitive to the presence of electrolytes. The electrolyte concentration can be reduced to a minimum by using ion exchange resins.

An example of commercial silica sols useful in the context of the present invention, are the Bindzil® series sold by Akzo Nobel AB.

*Particles of silica and "AgSol"*

A colloidal dispersion of particles of silica as described herein, including particles named "AgSol" comprise particles having a particle size from 3 nm to 25 nm and comprising silver ions on at least a part of a surface of said particles. Such colloidal dispersions may be prepared by contacting colloidal silica with a solution of silver nitrate ($AgNO_3$) in water. Notably, however, most soluble silver salts can be used to prepare the dispersion presented herein.

The particles of the present invention have a particle size from 3 nm to 25 nm, such as from 3 to 10 nm or from 3 to 5 nm.

The charge of metal ions in solution, usually aqueous solutions, is normally positive. This is the case for silver, which usually, but not always, forms monovalent cations in aqueous solutions. To achieve strong adsorption of metal ions on the surface of nano-sized carrier particles the electrical charge of the latter should be high but of opposite charge to that of the metal ions. The charge on the particles in colloidal silica or the particles of silica in an aqueous environment increases exponentially with pH and is almost 0.5 units of negative charge per $nm^2$ particle surface at pH of about 10 and at very low, $10^{-4}$ normal, electrolyte concentrations. Colloidal silica has a local stability maximum at the point of zero charge, which occurs at about pH 2.0. The stability of a silica sol first decreases with pH and reaches a minimum around pH 6, after which the sol enters a region of high stability between pH 8 and pH 10.5.

The surface charge of silica, and of many other metal oxides as well, can be altered by modifying the surface in different ways. In one method, when the particle surface of silica sols is modified with aluminosilicate ions (e.g. by treating sols with sodium aluminate solution) to create aluminosilicate sites on the particle surface, the surface will have a fixed, pH-independent negative charge that will make the sol more stable towards gelling by the presence of electrolytes and at low pH, for instance pH 4 to pH 5, than the sols from which they were prepared. Therefore, the particles of silica described herein may have aluminosilicate sites at the particle surface.

A convenient way to introduce aluminosilicate sites on the surface of colloidal silica particles is to use weak acid cation resin to remove sodium ions from the silica sol - sodium aluminate system and thus bring about reaction of the aluminate ions with the surface of the silica particle. In this system, pH will usually not fall below pH 5 even if an excess of weak acid cation exchange resin is used.

A calculated amount of sodium aluminate solution to give the desired number of aluminosilicate sites per $nm^2$ particle surface is simply added to the slurry of colloidal silica and resin. The creation of aluminosilicate sites on the surface of silica is also well described in the literature, (e.g. in Iler, The Chemistry of Silica, 1979, pp. 407-409). Such descriptions also indicate that it is difficult to introduce much more than about 2 aluminosilicate sites per $nm^2$ silica surface, for example. The concentration of aluminosilicate sites on the surface of preferred AgSol particles comprised herein falls in the range from about 0.20-2.0 site per $nm^2$, e.g. 0.30-1.50, or 0.3-1.25, or 0.4-1.0 site per $nm^2$, e.g. 0.4-0.8 site per $nm^2$.

The aluminosilicate sites carry a negative charge, which must be neutralized by counter ions, most often $Na^+$ ions. Modification of the silica surface with sodium aluminate converts the surface to a cation exchanger.

Although adsorption of metal cations on aluminosilicate-modified silica sols can be carried out over a wide pH range it is preferable to carry out the adsorption in the pH range where silica sols are most stable; that is the alkaline range, for instance in the pH range from about 8 to about 10.5. However, adsorption of metal cations on aluminosilicate-modified silica sols can be carried out over a wide pH range, e.g. from about pH of about 3 to a pH of about 12, e.g. from a pH of about 4 to a pH of about 11.5, or a pH of about 5 to a pH of about 11, e.g. a pH of about 6 to a pH of about 10.5.

The pH can be controlled at different steps of the process for making the composite sols. In most of the examples as described herein below, the silica sol was added to the transition metal salt solution and the pH was then adjusted to between 10 and 11 by adding 1 M NaOH-solution to the metal containing silica sol. Alternatively, alkali can be added to the silica sol before said sol is added to the metal salt solution or before the metal salt solution is added to said sol.

The rate at which silver salt solution can be added to the silica sol without destabilizing the sol depends on the conditions being used in the preparation. The rate of addition can be fast as long as the increments of added salt are virtually instantaneously dispersed throughout the sol and there rapidly adsorbed onto the silica particles. In many of the small scale preparations it is actually possible to inject 0.1 m $AgNO_3$ solution into magnetically stirred silics sols in very short times, for instance 10-15 seconds, without destabilizing the sols. However, in most of the small scale laboratory preparations, for instance preparations of sols containing about 500 ppm of metal, longer addition times of 0.1 molar silver salt solutions, typically 2-3 minutes were used so as to be on the safe side in terms of having good stability towards gelling or aggregation. Sols with higher silver contents may require longer times of addition. Thus, a sol containing 1500 ppm silver may require a time of addition of 0.1 molar silver solutions of about 12 minutes. Similar time scales will apply to larger scale preparations provided that agitation or stirring is as efficient as in the small scale preparations.

A sol of a given concentration of silver can be prepared in different ways. The reactants and products used in the various preparations and methods fall in the domain of colloids and colloid chemistry and due care has to be taken concerning concentration of reactants and products, maintaining a high electrical charge on colloidal particles, using water of good quality, preferably deionised water, observing proper rate of addition and order of addition of the components, working in conservative but realistic temperature ranges and providing sufficient agitation and stirring so as to maintain stability towards gelling or aggregation of reactants and products. Selecting and optimizing conditions of the before mentioned type are considered to be within the capacity of the person of ordinary skill in the art, in light of the present description and the embodying examples.

In one method, a certain amount of silver nitrate solution is added to a silica sol with specified values of particle size and concentration of silica. In another method, the same amount of silver nitrate solution is added to a sol of the same particle size but higher, for instance four times higher, concentration of silica. The overall concentration of silver is the same in the two sols but the concentration of silver on the particle surface of the former sol is higher - four times higher - than that of the latter sol. Thus, a colloidal dispersion presented herein with a given, overall concentration of silver and a given particle size may be obtained by combining high concentration of particles, that is a high concentration of silica, with a low concentration of silver on the particle surfaces or by combining high surface concentration of silver with low silica concentration.

The concentrations of silica of the sols used herein may vary from less than 0.1 % $SiO_2$ to 50 % $SiO_2$ preferably 0.5-30 % $SiO_2$, or more preferably 1-25 % $SiO_2$ and most preferably 2-10 % by weight of $SiO_2$, the remaining part (adding up to 100 %) comprising e.g. silver ions (species) and water.

The concentration of $SiO_2$ of the composite sol may also vary from about 0.5% by weight, or about 1% by weight, or about 2% by weight, to about 25 % by weight, or about 20 % by weight, or about 15 % by weight, or about 10 % by weight of $SiO_2$, or about 8% by weight, or about 5 % by weight, the remaining part (adding up to 100 %) normally comprising the selected metal ion(s) and water. The concentration of $SiO_2$ of the composite sol of the present invention may also range from about 0.005 % by weight to about 15% by weight, e.g. from about 0.1 % by weight to about 10 % by weight, or from about 0.5% by weight to about 5 % by weight.

An example of a colloidal dispersion presented herein comprises particles of silica to which silver ions have been adsorbed, wherein the silver ions are present in an amount of 0.05 ppm to 10 000 ppm by weight of the colloidal dispersion, such as 0.05 to 500 ppm, 0.05 to 200 ppm, 0.05 to 100 ppm, 0.05 to 50 ppm, 0.05 to 10 ppm, or 0.05 to 5 ppm by weight of the colloidal dispersion. The silver ions may also be present in an amount of 0.1 to 500 ppm, 0.1 to 200 ppm, 0.1 to 100 ppm, 0.1 to 50 ppm, 0.1 to 10 ppm or 0.1 to 5 ppm by weight of the colloidal dispersion.

The silver ions may also be present in an amount of 0.2 to 500 ppm, 0.2 to 200 ppm, 0.2 to 100 ppm, 0.2 to 50 ppm, 0.2 to 10 ppm, or 0.2 to 5 ppm by weight of the colloidal dispersion, or 0.3 to 500 ppm, 0.3 to 200 ppm, 0.3 to 100 ppm, 0.3 to 50 ppm, 0.3 to 10 ppm or 0.3 to 5 ppm by weight of the colloidal dispersion, or of 0.4 to 500 ppm, 0.4 to 200 ppm, 0.4 to 100 ppm, 0.4 to 50 ppm, 0.4 to 10 ppm or 0.4 to 5 ppm by weight of the colloidal dispersion.

The silver ions may also be present in an amount of 0.5 to 500 ppm, 0.5 to 200 ppm, 0.5 to 100 ppm, 0.5 to 50 ppm, 0.5 to 10 ppm or 0.5 to 5 ppm by weight of the colloidal dispersion, or 1 to 5000 ppm, 1 to 3000 ppm, 1 to 2000 ppm, 1 to 500 ppm, 1 to 200 ppm, 1 to 100 ppm, 1 to 50 ppm, 1 to 10 ppm or 1 to 5 ppm by weight of the colloidal dispersion or 5 ppm to 10 000 ppm by weight of the colloidal dispersion, such as 5 ppm to 10 000 ppm, 5 ppm to 5 000 ppm or 100 ppm to 5 000 ppm by weight of the colloidal dispersion.

*Silver population on particle surface*

Knowing the concentration of silver in the sol, the atomic weight of silver, the specific surface area of the silica particles and concentration of silica in weight percent, the surface concentration, $C_s$, of silver atoms (ions) per $nm^2$ of $SiO_2$ particle surface, can be calculated according to equation (1):

$$C_s = 60M_1/(M_{met}AK) = (60/AK)(M_1/ M_{met}) \qquad (1)$$

wherein

M$_1$ is the concentration of silver in the sol, in ppm,
M$_{met}$ is the atomic weight of the silver, in g,

A is the specific surface area of the sol particles, in m$^2$/g, and

K is the concentration of silica in weight percent

The concentration of silver ions on the surface of a preferred particle comprising a material used herein falls in the range from about 0.0005 (0.005) to more than 5 silver ions per nm$^2$, or from about 0.01 silver ion per nm$^2$ to more than 5 silver ions per nm$^2$, or from about 0.01 silver ions per nm$^2$ to about 2 silver ions per nm$^2$, e.g. about 0.01 to about 1 silver ion per nm$^2$, or about 0.05 to about 1 silver ion per nm$^2$, more preferably about 0.1 to about 0.8 silver ion per nm$^2$. Preferably, the concentration is 0.20 - 2.0, more preferably 0.50- 1.50, and even more preferably 0.70 - 1.25 ions (species) per nm$^2$.

In the case of silica particles having aluminosilicate sites at the surface, typically, one silver ion adsorbs on one charged Al-Si-site but not all Al-Si sites may have adsorbed silver species adsorbed on them. The ratio by number between silver ions and Al-Si sites may vary within 0.01 - 1.0, but is preferably between 0.05 - 0.8, e.g. between 0.1 and 0.6.

The load of silver ions vs. silica particles may be expressed either as number of silver cations per unit of surface area of the silica particles. This is the "specific silver load" or surface concentration of the silver cation $c_s$.

The load of silver ion vs. silica particles in the composite sol may also be expressed as the number of silver ions $n_m$ for each silica particle. However, for very low silver loads, it may be more meaningful to express the relationship between the number of silver ions and number of particles in the silica sol as the inverse of the number of silver ions for each silica particle, i.e. $n_m^{-1}$.

The relationship between $n_m^{-1}$ and $c_s$ is given by the equation (2):

$$n_m^{-1} = \frac{1}{n_m} * \frac{A_p}{A_p} = \frac{1}{c_s * A_p} \quad (2)$$

wherein

$n_m$ is the number of silver ions per silica particle in the composite sol,

Ap is the surface area of one silica particle in the composite sol, and

$c_s$ is the surface concentration of silver ions at the surface of the silica particle.

Equation (2) shows that $n_m^{-1}$ is inversely proportional to the surface area $A_p$ of the particle and the surface concentration $c_s$ of silver ions at the surface of the silica particles.

For $c_s$ = 0.0005 ions/nm$^2$, Table 1 illustrates how $n_m^{-1}$, viz. the number of silica particles per ion, varies as a function of the silica particle diameter.

Table 1. Number of silica particles per silver ion as a function of particle diameter in a composite sol, $c_s$ = 0.0005 ions/nm$^2$

| Particle diameter (nm) | number of particles per ion |
|---|---|
| 5 | 25 |
| 7 | 13 |
| 12 | 4.4 |
| 22 | 1.3 |

As may be seen from Table 1, at $c_s$ = 0.0005 ions/nm$^2$ and a particle diameter of 5 nm, 4 out of 100 silica particles in the composite sol of the invention carry a silver cation, viz. there are 25 particles present for each silver ion in the composite sol.

In other words, the number ratio between silver ions and silica particles in the composite sol of the present invention may vary from high values, where more than one silver ion is present for each silica nanoparticle, e.g. more than 10 silver ions are present for each silica nanoparticle, to low values, where more than one silica nanoparticle is present for each silver ion, e.g. more than 10 silica nanoparticles are present for each silver ion.

*Stability*

The term stable used herein may in some aspects means that the product should be stable toward gelling, implying that the relative viscosity should not increase more than 100 % (e.g. from 5 to 10 mPas) under a period of about two months. The term may also mean stability toward precipitation; i.e. there is no substantial precipitation of solid content, characterised by that no more than 20 % of the solid material has precipitated and settled as a sludge at the bottom, if stored under normal (e.g. ambient or optionally protected from light) conditions, for a period of two months.

As mentioned previously herein, although silica sols are stable over a wide pH range it is preferable to prepare the colloidal dispersions of the invention in the pH region of 8-12, more particularly 9-11, where silica sols are most stable.

*Further aspects of the invention*

As mentioned herein, there is a continuous need for improved treatments for bacterial infections, as well as for treatments that can serve as alternatives or at least serve to decrease the usage of traditional antibiotics.

Accordingly, the present invention relates to a pharmaceutical composition comprising an antibiotic agent according to the appended claims and a colloidal dispersion of particles of silica having a particle size from 3 nm to 25 nm to which particles silver ions have been adsorbed. An antibiotic agent in combination with a colloidal dispersion comprising particles of silica as described herein was shown to provide a synergistic antibacterial effect on a variety of different bacteria. As further explained herein, the antibiotic agent and the colloidal dispersion may be present in the same pharmaceutical composition or they may be administered as separate compositions. The particles have a particle size of 3 nm to 25 nm, such as 3 nm to 10 nm, such as 3 nm to 5 nm. Silver ions may be presented in an amount of 0.05 ppm to 50 ppm by weight of the dispersion such as 0.05 ppm to 5 ppm by weight of the colloidal dispersion, or as otherwise defined herein.

By "adsorption" is meant that the metal ion attaches to the surface, whether by electrostatic or ionic bonding or any other type of bonding, e.g. partly covalent bonding. The adsorption of metal ions on the surface of the silica particles may be monitored by measuring the Zeta potential of the colloidal sol.

The term "antibiotic agent" may be used interchangeably herein with the term "antibacterial agent", and may be defined as an agent having an inhibitory or preventing effect on the growth and/or survival of bacteria. Hence, an antibacterial or antibiotic agent can be bacteriostatic and/or bactericidal.

The expression "a colloidal dispersion of particles of silica having a particle size from 3 nm to 100 nm to which particles silver ions have been adsorbed" may also sometimes be referred to herein as a "colloidal dispersion", a "colloidal dispersion containing silver", or a "colloidal dispersion containing silver ions" or the like. Sometimes it is also simply referred to as "silica sol", "composite sol" or a "sol" herein.

Said antibiotic agent may be selected from the group of antibiotics named aminoglycosides. Aminoglycosides are well known in the art and comprise substructures of aminosugar and generally present bactericidal activity against Gram-negative aerobic bacteria. Examples of aminoglycosides for use herein comprise Streptomycin, Gentamycin, Tobramycin, Amikacin, Netilmicin, Neomycin, and Kanamycin, or a combination thereof. Particularly useful herein are Gentamycin and Tobramycin.

Said antibiotic agent may also be selected from the group of antibiotics named beta-lactams (β-lactams). Beta-lactams are a class of broad-spectrum antibiotics comprising bactericidal antibiotic agents containing a beta-lactam ring in their structure, and are also well-known. Examples of β-lactams for use herein include Ampicillin and Cefotaxime, or a combination thereof. Said antibiotic agent may also be selected from a steroid antibiotic, such as fusidic acid and/or mupirocin, but is not limited thereto.

In addition, said antibiotic agent may be a tetracycline, such as Tetracycline, Oxytetracycline, Doxycyclin and tigecycline.

Other examples of antibiotic agents include Rifampicin, Spectinomycin, Chloramphenicol and Erythromycin. Other examples of antibiotic agents include Vankomyin and Cefazolin.

The antibiotic agent according to the claimed invention is selected from the group as defined in the appended claims.

There is also provided herein a kit of parts comprising a pharmaceutical formulation as further defined herein including an antibiotic agent according to the appended claims, optionally in admixture with a pharmaceutically acceptable excipient; and a colloidal dispersion comprising particles of silica having a particle size from 3 to 25 nm, to which particles silver ions have been adsorbed. Examples of suitable pharmaceutically acceptable excipients are described elsewhere herein. Said pharmaceutical formulation may comprise more than one pharmaceutical excipient. The antibiotic agent used in said kit may be selected from aminoglycosides, beta-lactams, steroid antibiotics and tetracyclines examples of which previously have been mentioned herein and which are also applicable to the kit of parts described herein. The antibiotic agent according to the claimed invention is selected from the group as defined in the appended claims.

In a further aspect, there is provided a colloidal dispersion comprising particles of silica having a particle size from 3 nm to 25 nm to which particles silver ions have been adsorbed, for use in a therapeutic method for the treatment of a bacterial infection, by administration of said colloidal dispersion in combination with an antibiotic agent according to the appended claims.

There is also provided an antibiotic agent according to the appended claims for use in a therapeutic method for the treatment of a bacterial infection, by administration of said antibiotic agent in combination with a colloidal dispersion comprising particles of silica having a particle size from 3 nm to 25 nm to which particles silver ions have been adsorbed.

Accordingly, as mentioned elsewhere herein, the antibiotic agent and the colloidal dispersion comprising particles of silica may be administered separately, as separate compositions, or they may be present in the same pharmaceutical composition.

The therapeutically effective, or the pharmaceutically effective amount of the antibiotic agent in combination with the colloidal dispersion as presented herein depends e.g. on the species of mammal, e.g. a human or an animal, the body weight, the age, the individual condition, individual pharmacokinetic data, the disease to be treated and the mode of administration. Any use of a colloidal dispersion as described herein includes uses at least in human and veterinary medicine applications.

Equally, when said colloidal dispersion is presented for use in a method of treatment of a bacterial infection together with an antibiotic agent, or said antibiotic agent is presented for use in a method of treatment in combination with the colloidal dispersion, said antibiotic agent may be selected from aminoglycosides, beta-lactams, tetracyclines or steroid antibiotics according to the appended claims.

A bacterial infection may be an infection by gram positive bacteria or gram negative bacteria. An example of gram positive bacteria includes *Staphylococcous aureus.* Examples of gram negative bacteria include *Pseudomonas aeruginosa, Salmonella enterica, Escherichia coli,* and *Acinetobacter baumannii.*

Accordingly, a bacterial infection may be an infection by *Staphylococcous aureus, Pseudomonas aeruginosa, Salmonella enterica, Escherichia coli,* and/or by *Acinetobacter baumannii,* but is not limited thereto.

The bacteria may be sensitive or non-sensitive (i.e. resistant) to the antibiotic agent.

*Staphylococcous aureus* is part of the skin, nose and respiratory tract flora. It can cause a range of illnesses from mild skin infections to pneumonia, meningitis and sepsis. *Pseudomonas aeruginosa* is an opportunistic pathogen and it can cause infections in, for example, patients with cystic fibrosis and traumatic burns.

Preferably, the bacterial infection that is treated with the pharmaceutical composition as provided herein or with the colloidal dispersion in combination with an antibiotic agent as presented herein is a bacterial infection by *Staphylococcous aureus* and/or by *Pseudomonas aeruginosa.*

Notably, when a treatment of a bacterial infection is mentioned herein, such a treatment includes e.g. the treatment of a bacterial infection of the skin or of any mucosal surface.

The administration of the colloidal dispersion and the antibiotic agent, or the pharmaceutical composition as described herein, may be effected by local or systemic administration. Systemic administration may e.g. be effected via the oral, parenteral, rectal or pulmonary route. Local administration may e.g. be effected via the topical, oral, rectal or pulmonary route. Particularly, local administration to the intestines and the rectal area is envisaged herein. Furthermore, topical administration to the skin is also particularly envisaged.

Said administration comprises administering a therapeutically effective amount of the colloidal dispersion and the antibiotic agent, or the pharmaceutical composition optionally in association with a (i.e. one or more) pharmaceutically acceptable excipient, e.g. a pharmaceutically acceptable carrier.

For enteral, e.g. oral, administration, the pharmaceutical composition may be formulated in a wide variety of dosage forms. The pharmaceutically acceptable carriers may be either solid, semi-solid or liquid. Solid form preparations include powders, tablets, pills, lozenges, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The formulation may comprise an encapsulating material as carrier, providing a capsule in which the colloidal dispersion and the antibiotic agent, with or without carriers, is surrounded by a carrier, which is in association with it.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water with viscous material, such as natural or synthetic gums, resins, methylcellulose, sodium carboxymethylcellulose, and other well-known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The colloidal dispersion and the antibiotic agent, or the pharmaceutical composition may also be administered parenterally, e.g. by, injection or infusion, e.g. by intravenous, intraarterial, intraosseous, intramuscular, intracerebral, intracerebroventricular, intrasynovial, intrasternal, intrathecal, intralesional, intracranial, intratumoral, intracutaneous and subcutaneous injection or infusion.

Thus, for parenteral administration, the pharmaceutical compositions of the invention may be in the form of a sterile injectable or infusible preparation, for example, as a sterile aqueous or oleaginous suspension. This suspension may be formulated according to techniques known in the art using suitable dispersing or wetting agents (e.g., Tween 80), and suspending agents. The sterile injectable or infusible preparation may also be a sterile injectable or infusible solution or suspension in a non-toxic parenterally acceptable diluent or solvent. For example, the pharmaceutical composition may be a solution in 1,3-butanediol. Other examples of acceptable vehicles and solvents that may be employed in the compositions of the present invention include, but are not limited to, mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions.

These oil solutions or suspensions may also contain a long-chain alcohol diluent or dispersant.

Solutions for parenteral use also may contain suitable stabilizing agents, and if necessary, buffer substances. Suitable stabilizing agents include antioxidizing agents, such as sodium bisulfate, sodium sulfite or ascorbic acid, either alone or combined, citric acid and its salts and sodium EDTA. Parenteral solutions may also contain preservatives, such as benzalkonium chloride, methyl- or propyl-paraben, and chlorobutanol.

For inhalation (pulmonal or nasal administration), suitable pharmaceutical formulations are as particles, aerosols, powders, mists or droplets, e.g. with an average size of about 10 $\mu$m in diameter or less. For example, compositions or formulations for inhalation may be prepared as solutions in saline, employing benzyl alcohol or other suitable preservatives, absorption promoters to enhance bioavailability, fluorocarbons, and/or other solubilizing or dispersing agents known in the art.

The pharmaceutical compositions, or the antibiotic agent and the colloidal dispersion of particles of silica as separate compositons, of the invention also may be administered topically, to the skin or to a mucous membrane. For topical application, the pharmaceutical composition, or the antibiotic agent and the colloidal dispersion as separate compositions, may be e.g. a lotion, a cream, a gel, a paste, a tincture, a transdermal patch, a spray or a gel for transmucosal delivery. The pharmaceutical compositions, or the antibiotic agent and the colloidal dispersion as separate compositions may be in the form of a solution or suspension. The composition, or the antibiotic agent and the colloidal dispersion as separate compositions may be formulated with a suitable ointment containing the active components suspended or dissolved in a carrier. Carriers for topical administration of the pharmaceutical composition, or the antibiotic agent or the colloidal dispersion as separate compositions of this invention include, but are not limited to, mineral oil, liquid petroleum, white petroleum, propylene glycol, polyoxyethylene polyoxypropylene compound, emulsifying wax and water. Alternatively, the pharmaceutical composition, or the antibiotic agent and the colloidal dispersion as separate compositions may be formulated as a suitable lotion or cream containing the active compound suspended or dissolved in a carrier. Suitable carriers include, but are not limited to, mineral oil, sorbitan monostearate, polysorbate 60, cetyl esters wax, cetaryl alcohol, 2-octyldodecanol, benzyl alcohol and water. The pharmaceutical compositions, or the antibiotic agent and the colloidal dispersion as separate compositions of this invention may also be topically applied to the lower intestinal tract by rectal suppository formulation or in a suitable enema formulation.

Suitable pharmaceutical excipients, e.g. carriers, and methods of preparing pharmaceutical dosage forms are described in Remington's Pharmaceutical Sciences, Mack Publishing Company, a standard reference text in art of drug formulation.

In general, the pharmaceutical composition or the colloidal dispersion and the antibiotic agent will be administered in a therapeutically effective amount, or a pharmaceutically effective amount by any of the accepted modes of administration for agents that serve similar utilities. Suitable daily dosages typically ranges from 1 to 1500 mg, e.g. 1-500 mg daily, or 1-50 mg daily, but will also depend upon numerous factors such as the severity of the disease to be treated, the age and relative health of the patient, the route and form of administration, and the indication towards which the administration is directed, etc. One of ordinary skill in the art of treating such diseases will be able, without undue experimentation and in reliance upon personal knowledge and the disclosure of this application, to ascertain a therapeutically effective amount of the pharmaceutical composition or the colloidal dispersion and the antibiotic agent presented herein for a given disease. As an example, it is envisaged that the colloidal dispersion can be administered in an amount of about 0.1 to 0.5 of MIC Ag. It is envisaged that the dose of the antibiotic agent is reduced when the antibiotic agent is administered in combination with the colloidal dispersion presented herein.

Particularly, it is envisaged that administration is effected by the topical route, such as in the form of a paste, lotion, cream, spray or an ointment. Administration can also be effected through a patch, or the like, to which a pharmaceutically effective amount of the colloidal dispersion and an antibiotic agent, or the pharmaceutical composition as described herein has been absorbed. Furthermore, it may be suitable to administer the colloidal dispersion and the antibiotic agent, or the pharmaceutical composition as described herein orally, or even more preferably by the pulmonary route (e.g. by inhalation), such as by using a pulmonary drug delivery platform such as a pressurized metered-dose inhaler (pMDI), Dried-Powder Inhaler (DPI), aqueous metered-dose inhaler (MDI)/small volume nebulizer, a Soft Mist Inhaler or the like, or be given by nebulisation, such as by using a nasal spray of an inhalation device.

Particularly, bacterial infections of wounds, lesions or sores, such as diabetes wounds and burns, or the like, caused e.g. by *Staphyloccous aureus, Pseudomonas aeruginosa, and/or Acinetobacter baumannii* may advantageously be treated via the topical route e.g. by using a paste, cream, lotion or an ointment comprising the pharmaceutical composition or the colloidal dispersion in combination with the antibiotic agent as provided herein, or by using a spray making it possible to partly or fully cover the wound to prevent spread of the bacterial infection.

In particular aspects, there is provided a pharmaceutical composition or a colloidal dispersion as described herein in combination with an antibiotic agent for use in the treatment of a bacterial infection of *Pseudomonas aeruginosa* wherein said antibiotic agent is an aminoglycoside, such as Streptomycin, Gentamycin, Tobramycin, Amikacin, Netilmicin or Kanamycin, or a combination thereof. In one aspect, Gentamycin and/or Tobramycin are used in combination with a pharmaceutical composition or a colloidal dispersion comprising silver ions as disclosed herein.

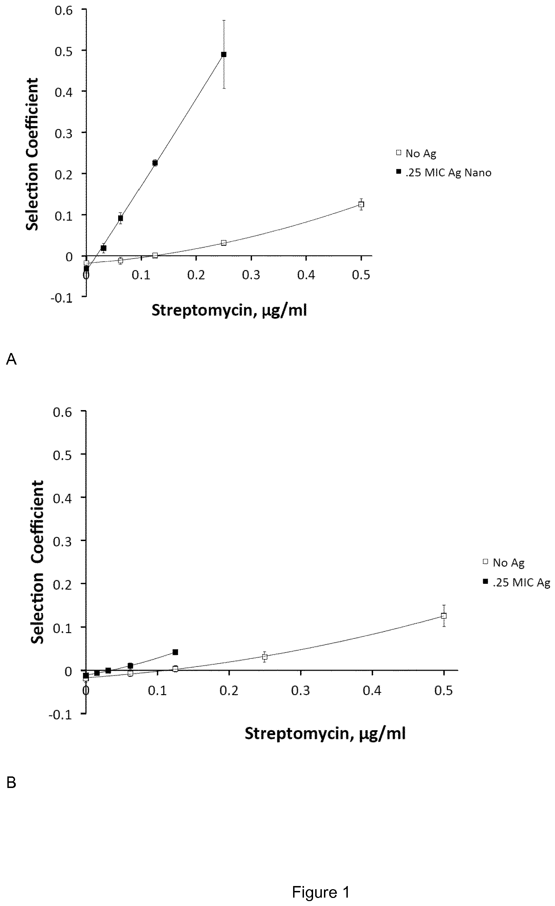

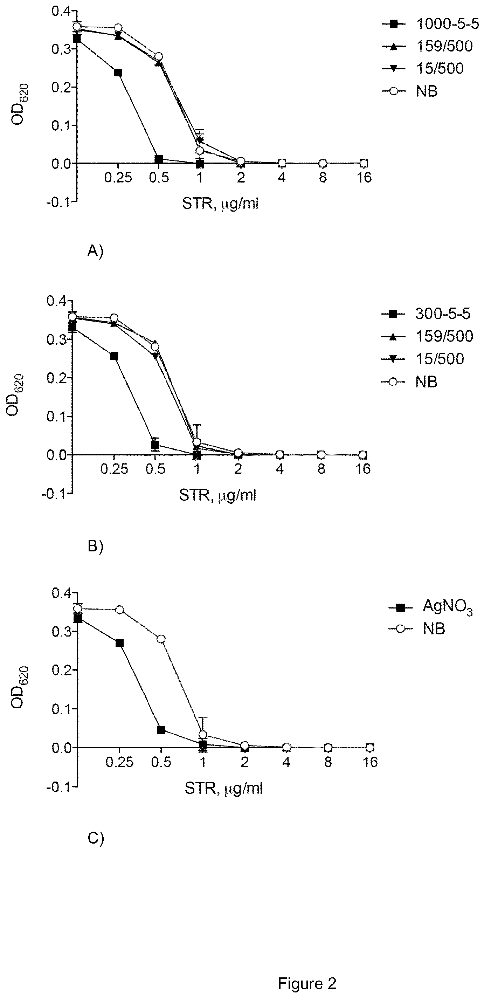

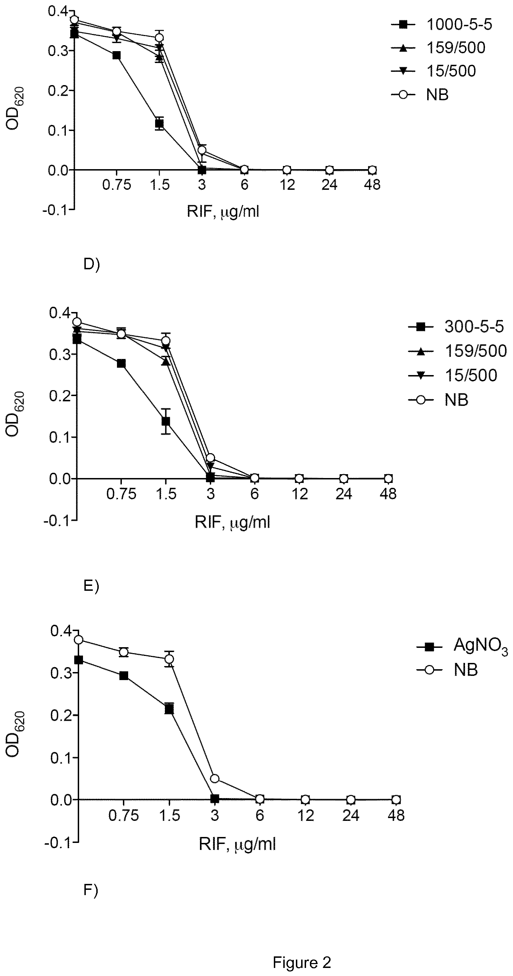

Particularly, an antibacterial synergistic effect was shown on *Salmonella enterica* cultures when an aminoglycoside (e.g. Streptomycin) was used as the antibiotic agent in combination with the colloidal dispersion comprising silver ions (see Figures 1 and 2). This data supports a synergistic antibacterial effect of an aminoglycoside and a colloidal dispersion comprising silver ions on gram negative bacteria.

Hence, it is an object of the present invention to provide a pharmaceutical composition or a colloidal dispersion of silica particles having a particle size from 3 nm to 25 nm to which particles silver ions have been adsorbed in combination with an antibiotic agent according to the appended claims, which combination is capable of providing a synergistic antibacterial effect resulting in an improved antibacterial effect.

However, there is also an object to provide a pharmaceutical composition or a colloidal dispersion as presented herein in combination with an antibiotic agent, which combination is capable of providing a synergistic anti-bacterial effect. As an example, a pharmaceutical composition or a colloidal dispersion in combination with an antibiotic agent as provided herein surprisingly facilitates the usage of reduced amounts of antibiotic agent when effected through the combination of said colloidal dispersion and an antibiotic agent.

As previously mentioned, there is a constant need to identify pharmaceutical compositions that can be used as alternatives to traditional antibiotics. Surprisingly, in that context, the colloidal dispersions in combination with an antibiotic agent were proven a very useful alternative to traditional antibiotics by themselves.

In more specific aspects, said antibiotic agent is an aminoglycoside, such as Streptomycin, Gentamycin, Tobramycin, Amikacin, Netilmicin, or Kanamycin, or a combination thereof, more particularly Gentamycin and/or Tobramycin and the bacterial infection is a gram-negative (aerobic) bacterial infection. In yet another more specific aspect, the gram-negative (aerobic) bacterial infection is an infection with *Pseudomonas aeruginosa.*

It was shown that a strong synergistic antibacterial effect was obtained using a colloidal dispersion as presented herein in combination with an aminoglycoside on gram-negative bacteria. This is described in example 3 and shown in Table 6. Therefore, in a more specific aspect, there is provided herein a pharmaceutical composition comprising an aminoglycoside, such as Gentamycin and/or Tobramycin, in combination with a colloidal dispersion for use in the treatment of a bacterial infection of gram negative bacteria, such as *Pseudomonas aeruginosa* or *Escherichia coli.* Importantly and surprisingly, a 15-17 fold increase in effect was seen when Tobramycin or Gentamycin was used. Such an increase in effect implies or suggests that lower doses of antibiotic agents may be used to achieve an antibacterial effect when such antibiotic agents are combined with the herein described colloidal dispersions as compared to when only an antibiotic agent is used.

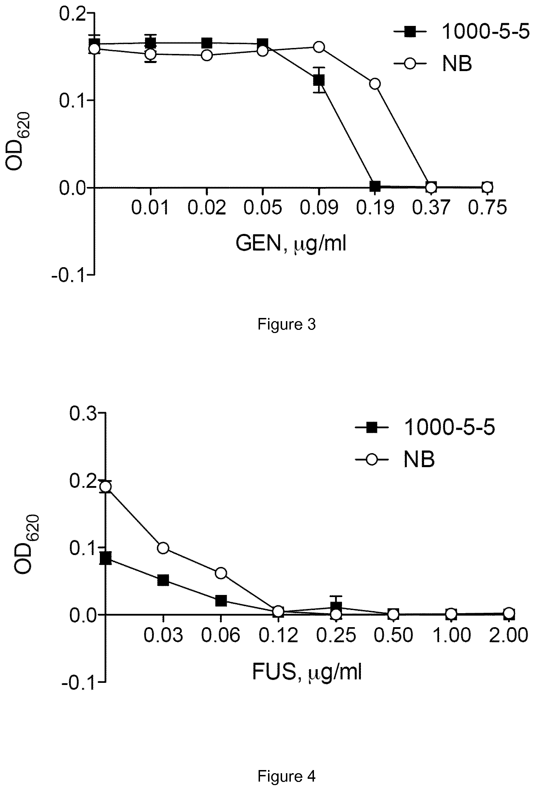

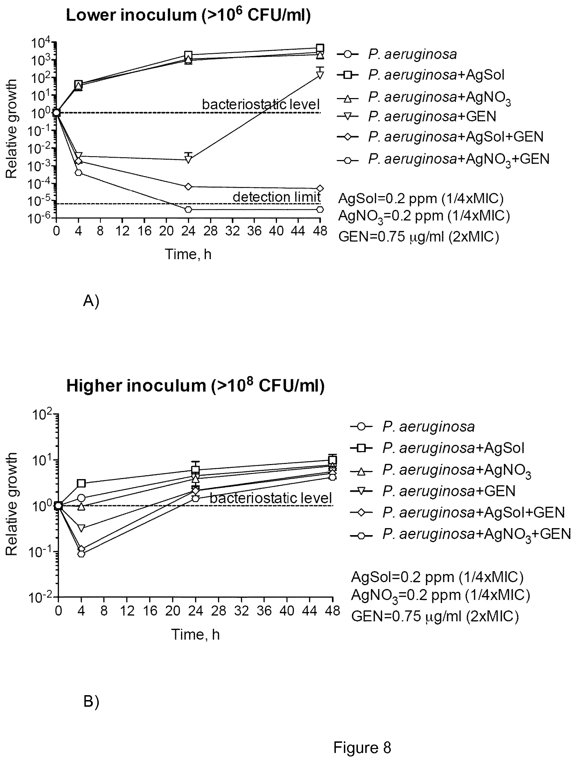

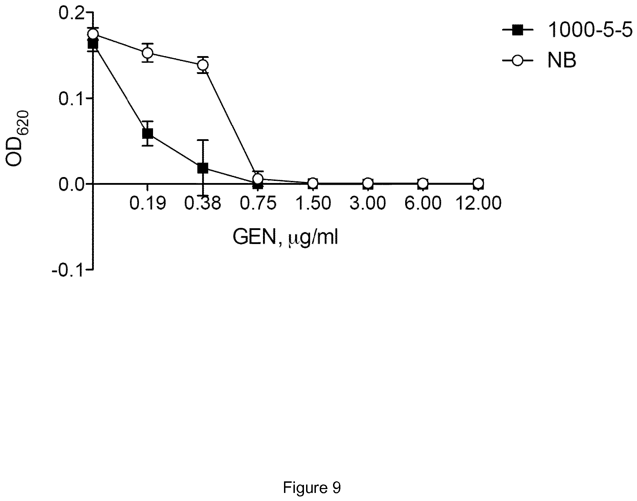

A synergistic anti-bacterial effect on *Pseudomonas aeruginosa* was obtained using the colloidal dispersions presented herein in combination with an aminoglycoside (Gentamycin). The results are further shown in examples 4 and 6 and figures 3 and 8 and in figure 9. Therefore, there is provided particularly herein a pharmaceutical composition or a colloidal dispersion containing silver ions as described herein in combination with an aminoglycoside, such as Gentamycin and/or Tobramycin, for use in treating a bacterial infection with *Pseudomonas aeruginosa.* Preferably, said treatment is effected through topical administration. Examples of topical administration modes have previously been presented herein.

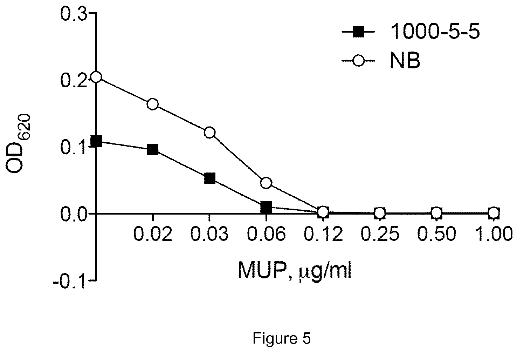

In another aspect, said antibiotic agent is a steroid antibiotic, e.g. fusidic acid (FUS) and/or mupirocin (MUP) and the bacterial infection is an infection with *Staphylococcus aureus. A* synergistic anti-bacterial effect on *Staphylococcus aureus* was shown using the colloidal dispersion presented herein in combination with a steroid antibiotic, such as fusidic acid (FUS) and/or mupirocin (MUP). The effect obtained is further described in example 4 and the results are illustrated in figure 4 and 5. Therefore, there is provided particularly herein a pharmaceutical composition or a colloidal dispersion in

EP 3 727 449 B1

combination with a steroid antibiotic, such as fusidic acid (FUS) and/or mupirocin (MUP), for use in treating an infection with *Staphylococcus aureus.* It is particularly envisaged that a *Staphylococcus aureus* infection is treated by topical administration of the pharmaceutical composition. It is further envisaged that the treatment is a treatment of a skin infection. Fusidic acid (FUS) may be administered intranasally.

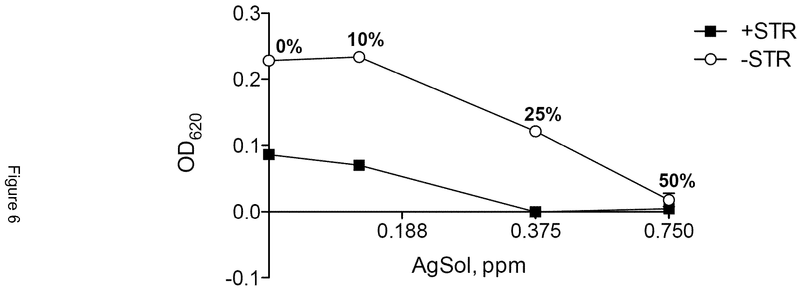

In example 5, the content of AgSol was varied to 0%, 10%, 25%, and 50% of MIC (AgSol) while the content of streptomycin was kept constant. Notably, an effect on gram negative bacteria (*Escherichia coli*) was seen already at 10% of MIC of AgSol (Figure 6). Therefore, it is envisaged that an antibacterial effect can be achieved by a pharmaceutical composition or a combination as disclosed herein using less antibiotic agent than when the antibiotic agent is used alone. The amount of antibiotic agent to be used in this context will vary depending on the antibiotic agent used.

There is further provided herein the colloidal dispersion for use in a method of treatment of a bacterial infection, or the antibiotic agent for use in a method of treatment of a bacterial infection, wherein the colloidal dispersion and the antibiotic agent are administered in sequence or concurrently.

There is also provided herein an antibiotic agent for use in a method for the treatment of a bacterial infection, wherein said treatment is by administration in combination with a colloidal dispersion comprising particles of silica having a particle size from 3 nm to 25 nm to which particles silver ions have been adsorbed. As mentioned elsewhere herein, the antibiotic agent may be selected from the group as defined in the appended claims. The bacterial infection may be an infection by gram positive or gram negative bacteria. Examples of gram negative bacteria include *Pseudomonas aeruginosa, Salmonella enterica, Escherichia coli,* and *Acinetobacter baumannii.* The antibiotic agent and the colloidal dispersion may be administered separately, concurrent or subsequently, or may be present together in a pharmaceutical composition.

The invention will now be further illustrated and exemplified by the following experimental section, but is not intended to be limited thereto.

EXPERIMENTAL SECTION

Materials and methods

"Highly sensitive competition experiments"

*Minimum Inhibitory Concentration*

The MIC (Minimal Inhibitory Concentration) of a bacterium to a certain antimicrobial agent gives a quantitative estimate of the susceptibility. MIC is defined as the lowest concentration of antimicrobial agent required to inhibit growth of the organism. The principle is simple: Agar plates, tubes or microtitre trays with two-fold dilutions of antibiotics are inoculated with a standardized inoculum of the bacteria and incubated under standardized conditions following NCCLS guidelines. The next day, the MIC is recorded as the lowest concentration of antimicrobial agent with no visible growth. The MIC informs you about the degree of resistance and might give you important information about the resistance mechanism and the resistance genes involved. MIC-determination performed as agar dilution is regarded as the gold standard for susceptibility testing (Source: Global Salm-Surv - A global Salmonella surveillance and laboratory support project of the World Health Organization (4th Ed. April 2002, modified Nov 2010))

The Minimum Inhibitory Concentration (MIC) of nanosilver (silver) was determined by two-fold dilution series in NaCl free Nutrient Broth (Sigma-Aldrich) in 15 ml screw cap tubes. The tubes were incubated in the dark over night at 37°C without shaking. The MIC was determined as the lowest concentration of nanosilver (silver) at which no visible growth was observed.

*Competition experiments*

The competing strains were a *Salmonella enterica Typhimurium* LT2 encoding antibiotic resistance (rpoB S531L for rifampicin and rpsL105 for streptomycin) against the isogenic counterpart lacking the resistance cassette, referred to as wild type. Competitions were performed in a series of sub-inhibitory concentrations of antibiotic (rifampicin or streptomycin) with and without nanosilver at $0.25 \times MIC_{nanosilver.(silver)}$ To track the two strains, the resistant strain was labeled with yellow fluorescent protein (YFP) and the wild type with blue fluorescent protein (BFP). A pair with the opposite configuration of tags was also created. The strains were grown over night in NaCl free Nutrient Broth (Sigma-Aldrich). The following day, the resistant strain and the wild type were mixed at a 1:1 ratio, and $10^6$ CFU/ml were added to two sets of tubes: one containing a series of sub-inhibitory concentrations of the antibiotic, and the other containing an identical series of sub-inhibitory antibiotic concentrations in addition to a constant concentration of nanosilver (silver) ($0.25 \times MIC_{nanosilver (silver)}$). The samples were incubated for 18-20 hours at 37°C with aeration. After incubation, 1 µl of each culture ($10^6$ CFU) was passaged to 1 ml of newly prepared solution (a 1:1000 dilution which corresponds to every ten generations).

Concurrent with the passaging, the ratio of resistant strain to wild type was measured by counting 105 cells using flow cytometry (BD Biosciences; BD FACSAria Ilu). The selection coefficients were calculated using the regression model s = [ln[R(t)/R(0)]]/t as described by Dykhuizen, where R is the ratio of resistant strain to wild type.

Reference

Dykhuizen, Daniel E, Experimental Studies of Natural Selection in Bacteria, Annual Review of Ecology and Systematics, Vol. 21 (1990), pp. 373-398

"AgSol-antibiotic synergy screening assay"

Antibiotic stock solutions were serially diluted in 2-fold steps in pre-warmed (37 °C) nutrient broth (NB) to prepare an antibiotic concentration gradient above and below the minimal inhibitory concentration (MIC) of antibiotic (Table 4). 50 $\mu$l of prepared dilutions were aliquoted into a 96-well round bottom plate (Nunc, Thermo Fisher) for the screening assay.

Silver formulation (AgSol1000 (particles with 19 Ag per silica particle), AgSol300 (particles with 6 Ag per silica particle, AgNO$_3$), aluminum coated silica beads (159/500 (silica particles with aluminium silicate coating)) and uncoated silica beads (15/500) were diluted in pre-warmed (37 °C) NB. Bindzil® 15/500 is an unmodified silica sol, whereas Bindzil® 159/500 is a aluminosilicate modified silica sol. Bindzil® is sold by Akzo Nobel AB.

Table 2. Characterization of Bindzil® silica sols

| Bindzil® | Specific surface area m$^2$/g | Part. size nm | Silica % | pH | Viscosity cp | Density | Na$_2$O % by weight | Al$_2$O$_3$ % by weight |
|---|---|---|---|---|---|---|---|---|
| 15/500 | 525 | 5 | 15 | 10.0 | 3 | 1.1 | 0.4 | - |
| 159/500 | 525 | 5 | 15 | 9.5 | 3.5 | 1.1 | 0.4 | 0.5 |

Table 3. Characterization of AgSol1000 and AgSol300 (values in Table are approximate)

| | Particle size, nm | Silica conc. % | Aluminosilicate sites per nm$^2$ | pH of starting sol | Amount of 0.1 m AgNO$_3$ solution, g | Time of addition, minutes | Concentr. of ads. metal in sol, ppm | pH of composite sol |
|---|---|---|---|---|---|---|---|---|
| AgSol1000 | 5 | 5 | 0.7 | 10-11 | 5.1 | 5 | 1000 | About 9-10 |
| AgSol300 | 5 | 5 | 0.7 | 10-11 | 1.7 | 2.5 | 300 | About 9-10 |

40 $\mu$l of the prepared dilutions were added to the 96-well plate containing antibiotic gradient to reach the final silver concentration of 1/4xMIC$_{AgSol\ (silver)}$ (specific for the microorganism used) (Table 5).

Overnight (16-20 h) cultures of 3 biological replicates of the following microorganisms *E. coli* K-12 MG1655 (DA4201), *E. coli* K-12 MG1655 *rpsL* K42N (DA49828), *Pseudomonas aeruginosa* PAO1 (DA6215) or *Staphylococcus aureus* USA300 JE2 (DA28823) were grown in NB till saturation (37 °C incubator with 190 rpm shaking). Cultures were diluted in NB to 10$^7$ CFU/ml and 10 $\mu$l of the dilution were added to the 96-well plate containing antibiotic gradient and silver formulations or the bead controls to reach the final concentration of 10$^5$ CFU/well in 100 $\mu$l total volume per well. 3 technical replicates (3 wells) of specific antibiotic concentration in combination with silver and microorganism were used. A control plate containing bacterial cells mixed with only antibiotic gradient was always included too.

The prepared plate was incubated in 37°C incubator without shaking in the dark for 16-20 h and the optical density (OD$_{620}$) of each well was measured in plate reader Multiscan FC (Thermo Fisher). Mean OD$_{620}$ and standard deviation of 3 biological replicates was plotted as a function of antibiotic concentration gradient with or without silver treatment to determine, if addition of silver to antibiotic gradient had an effect on the growth of the microorganism and antibiotic MIC.

"Time-kill assay"

Antibiotic stock solutions were diluted in pre-warmed (37°C) nutrient broth (NB) to prepare 2xMIC concentration (Table 4) of antibiotic in the final volume of 2 ml in 15 ml tubes (Sarstedt).

Silver formulation (AgSol1000, AgNO$_3$) was diluted in pre-warmed (37°C) NB and added to the tube containing antibiotic to reach the final silver concentration of 1/4xMIC$_{AgSol\ (silver)}$ (Table 5) in the final volume of 2 ml.

Overnight (16-20 h) cultures of 2 biological replicates of the following microorganisms *Pseudomonas aeruginosa* PAO1 (DA6215) or *Staphylococcus aureus* USA300 JE2 (DA28823) were grown in NB till saturation (37°C incubator with 190 rpm shaking). Cultures were diluted (1/100) in pre-warmed (37 °C) NB and incubated in 37°C incubator with 190 rpm shaking until they reached exponential growth phase (OD$_{600}$ ~ 0.2). Final concentration of approximately 10$^6$ CFU/ml of exponentially dividing cells was used in the total volume of 2 ml mixture of antibiotic and silver formulation. Control tubes with only bacterial cells or bacterial cells mixed with only antibiotic or only silver formulation were also used.

All tubes were incubated in 37°C water bath with 150 rpm shaking and 100 μl samples were taken out at pre-determined time points for viable counts (2 technical replicates). CFU counts were normalized to the 0 h sample and were plotted as a function of time (h).

Table 4. MIC values used for antibiotic gradient in 96-well plate.

| Microorganism | Antibiotic | MIC (μg/ml) |
|---|---|---|
| *E. coli* (DA4201) | Streptomycin (STR) | 0.5 |
| | Kanamycin (KAN) | 0.5 |
| | Spectinomycin (SPC) | 12 |
| | Tobramycin (TOB) | 0.75 |
| | Gentamycin (GEN) | 0.5 |
| | Rifampicin (RIF) | 3 |
| | Chloramphenicol (CAM) | 3 |
| | Tetracycline (TET) | 1 |
| | Ampicillin (AMP) | 4 |
| | Cefotaxime (CTX) | 0.094 |
| | Erythromycin (ERY) | 48 |
| | Ciprofloxacin (CIP) | 0.012 |
| *E. coli* (DA49828) | Streptomycin (STR) | 2000 |
| *P. aeruginosa* (DA6215) | Gentamycin (GEN) | 0.375 |
| *S. aureus* (DA28823) | Fusidic acid (FUS) | 0.12 |
| | Mupirocin (MUP) | 0.12 |

Table 5. SubMIC (1/4xMIC) silver concentration used in the assay.

| Microorganism | AgSol MIC (ppm) | Final conc. (1/4xMIC) (ppm) |
|---|---|---|
| *E. coli* (DA4201) | 1.5 | 0.375 |
| *E. coli* (DA49828) | 0.8 | 0.2 |
| *P. aeruginosa* (DA6215) | 0.8 | 0.2 |
| *S. aureus* (DA28823) | 50 | 12.5 |

Results

Example 1

When the "highly sensitive competition experiments" were performed, i.e. utilizing a mixture of 50% *Salmonella enterica Typhimurium* bacteria that are sensitive (non-resistant to Streptomycin) and 50% bacteria that are resistant to Streptomycin, there was a synergistic effect when AgSol (AgNano) was used in combination with Streptomycin (Figure 1A). In addition, the synergistic effect of Streptomycin and AgSol (AgNano) was higher than the synergistic effect of AgNO$_3$ and Streptomycin (Figure 1B).

Example 2

When the "AgSol-antibiotic synergy screening assay" was used, an improved effect on *Salmonella* was seen when AgSol (1000-5-5 and 300-5-5; nanosilver with 19 g Ag per silica and 6 Ag per silica particle, respectively) or AgNO$_3$ was used in combination with two different antibiotics as compared to silica particles with Aluminium silicate coating and silica particles only (159/500 and 15/500, respectively). See figures 2A-2F. A stronger synergetic effect was seen for Streptomycin (STR) in combination with silver, but a synergetic effect was evident also for Rifampicin (RIF). The synergistic effect for AgSol in combination with Streptomycin was more prominent than for $AgNO_3$. In light of the sensitivity of this particular assay, it was not expected to see a difference in effect between AgSol and $AgNO_3$ in combination with an antibiotic agent. Therefore, these results add additional support to AgSol being capable of providing an improved synergistic effect as compared to $AgNO_3$ when provided in combination with an antibiotic agent, at least when used in combination with an aminoglycoside, such as Streptomycin.

*Example 3 (Ag/Antibiotic screen)*

A synergistic antibacterial effect was seen with AgSol (using different concentrations of Ag on surface of silica particles) in combination with antibiotics on *E.coli* (MG 1655 K-12), as compared to when only antibiotics was used (Table 6). The results also show a markedly improved synergistic effect for AgSol in combination with Streptomycin on *E.coli.*
For Kanamycin, Ampicillin and Cefotaxime, a similar synergetic effect on *E.coli* was shown when effected with AgSol in combination with the antibiotic agent as compared to when only antibiotic agent was used. For Tobramycin, Gentamycin and Streptomycin, there was a 16- and 17-fold increase in effect, respectively.

Table 6: Ag/Antibiotic screen (tetracycline, ciprofloxacin and erytrhomycin are reference antibiotics)

| *Escherichia coli* MG1655 K-12 | | | |
|---|---|---|---|
| | | | |
| **Antibiotic** | **Antibiotic MIC ($\mu$g /ml)** | | **Fold change** |
| | **NB + AgSol1000** | **NB** | |
| Streptomycin | 0.03 | 0.5 | 17x |
| Gentamicin | 0.03 | 0.5 | 17x |
| Tobramycin | 0.047 | 0.75 | 16x |
| Kanamycin | 0.12 | 0.5 | 4x |
| Spectinomycin | 6 | 12 | 2x |
| Ampicillin | 2 | 4 | 2x |
| Cefotaxime | 0.047 | 0.094 | 2x |
| Chloramphenicol | 1.5 | 3 | 2x |
| Rifampicin | 3 | 3 | - |
| Tetracycline | 0.5 | 0.5 | - |
| Ciprofloxacin | 0.012 | 0.012 | - |
| Erythromycin | 48 | 48 | - |

*Example 4*

Tests have been performed with P. *aeruginosa* (PAO1) with the antibiotic agent gentamicin (GEN). A synergistic effect (-50%) was seen for gentamicin in combination with AgSol (Figure 3) although the effect was not as significant as for *E.coli* (Table 4). An interesting observation was that at the effective concentration for gentamicin with silver, there was a very small effect actually provided by gentamicin (see e.g. the difference in $OD_{620}$ at 0.19ug/ml). This means that at lower concentrations of gentamicin, the combination with silver leads to a much better efficacy, e.g. at a concentration of gentamicin of 0.19ug/ml. There is a complete killing of bacteria with a combination silver/ gentamicin whereas almost no reduction in $OD_{620}$ has occurred.
For *S. aureus* (USA300 JE2), there was an effect for fusidic (FUS) acid and mupirocin (MUP) when adding AgSol (Figure 4 and 5). The effect was however less prominent than for *E.coli.* At the effective concentration for fusidic acid and mupirocin with silver, only a small part of the effect is the effect provided by the antibiotic agent. This shows that the presence of silver clearly influences the effect of the antibiotics on the bacteria. In addition to showing a higher effect, the data shows the possibility to obtain a reduction/killing of bacteria at a lower concentration for the combination silver/-antibiotic agent as compared to the antibiotic agent itself. i.e. a possibility to reduce the antibiotic burden and thus possibilities to reduce doses to patients/animals to reduce side effects while having the same effect on the reduction of bacteria.

*Example 5*

The ratio of Agsol was varied to 0%, 10%, 25% and 50% of MIC (Minimal Inhibitory Concentration) of AgSol with a constant ratio of streptomycin (+STR). *E.coli* (sensitive, not resistant) was used. There is a clear effect already at 10% of MIC by Agsol as compared to the same concentration of silver without the addition of streptomycin (-STR). At MIC 50% we see, as expected, an almost complete killing of the bacteria (Figure 6). The data provides evidence of the bacteria reducing effect of silver provided by itself and in combination with the antibiotic agent. The data provides the opportunity to reduce the concentration (dose) of an antibiotic agent while maintaining the effect on bacteria by adding a low (10-50%) concentration of AgSol to the antibiotic.

*Example 6*

A "Time Kill" test was performed with *S.aureus* USA3000 JE2 and Fusidic acid (FUS) and Mupirocin (MUP) and with *P. aeruginosa* (PAO1) with gentamicin (GEN). From a stock solution with bacteria with controlled growth at 37°C during 16 h, 4 tubes were prepared:

- Bacterial cells
- Bacterial cells + AgSol
- Bacterial cells + $AgNO_3$
- Bacterial cells + AgSol + antibiotic
- Bacterial cells + $AgNO_3$ + antibiotic

Samples were taken after 0, 4, 8, 24 and 48 hours at 37°C (incubation with shaking). The samples were plated for viable counts, CFU counts normalized to 0 h and plotted as a function of time.

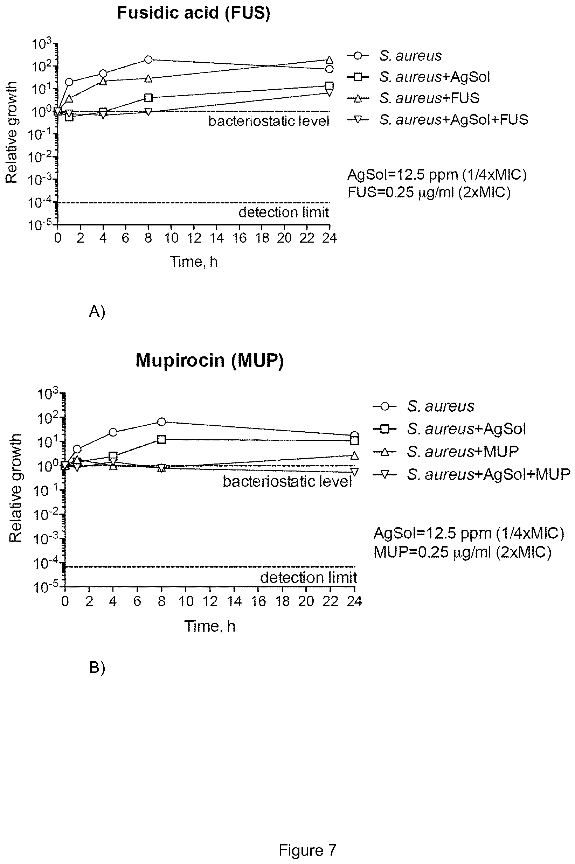

For *S.aureus* USA3000 JE2 and Fusidic acid (FUS) and Mupirocin, both antibiotic agents in combination with AgSol acted bacteriostatic (stopping the growth), see Figure 7A-B.

For *P. aeruginosa* (PAO1) with gentamicin (GEN), there was a bactericidal effect on *P.aeruginosa* cells. An even stronger bactericidal effect was observed when Agsol was used in combination with GEN. When the higher inoculum is used, the killing is less pronounced and the regrowth of cells happens after 48h. See Figure 8A and 8B.

For AgSol, there was a complete inhibition or killing of bacterial growth, which was not the case when gentamicin only was used. For gentamicin, it was evident that without the presence of silver, there is no complete inhibition or killing of bacterial growth. Instead, we see an increase in bacterial growth (in line with previous data). When an antibiotic agent is added, there is a bactericidal effect. Again, there was an almost complete killing (bactericidal effect) within 24 hours when gentamicin was used in combination with AgSol and no bacterial growth is visible within 48 hours. In contrast, when gentamicin only was used, there was a steady bacterial growth during the 48 hours that measurements were made.

*Example 7.1*

Tests will be performed with a resistant strain of *P.aeruginosa* with the antibiotic agent gentamicin (GEN) and *P.aeruginosa* with the antibiotic agent gentamicin (GEN) and AgSol as described in example 4 to confirm the effect of the combination of an antibiotic agent gentamycin and AgSol on a strain of *P.aeruginosa* (PAO1) resistant to antibiotics.

*Example 7.2*

As mentioned in example 7.1, a test was performed with a gentamicin-resistant strain of *P.aeruginosa* (DA60255) as described in example 4. The MIC of AgSol1000 for DA60255 was approximately 1.6 ppm and 0.25 x MIC of the concentration was used to evaluate its effect on gentamicin resistance. The test confirmed the anti-bacterial effect of AgSol and gentamycin (GEN) on a strain resistant to antibiotics. The results are shown in Figure 9.

*Example 8*

Tests will be performed to test the effect on *P.aeruginosa* (sensitive and resistant) by varying the amount of silver (AgSol) in the composition.

The following will be tested:

- Only AgSol in an amount of 5%, 10% or 25% of MIC (Minimal Inhibitory Concentration) of AgSol (Ag);

- Only Gentamycin (1/4);
- A combination of Gentamycin and AgSol (in varying amounts of 5%, 10% or 25% of MIC (Minimal Inhibitory Concentration) of AgSol (silver)).

The time course for performing example 8 will be about 3-4 weeks. A similar example, from which the basics details for this experiment can be retrieved, is found in Example 5.

**Claims**

1.  A pharmaceutical composition comprising:

    a) an antibiotic agent; and,
    b) a colloidal dispersion of particles of silica having a particle size from 3 nm to 25 nm to which particles silver ions have been adsorbed,
    wherein the antibiotic agent is selected from the group consisting of aminoglycosides, beta-lactams, steroid antibiotics, spectinomycin, chloramphenicol and rifampicin.

2.  The composition of claim 1, wherein the antibiotic agent is an antibiotic of the group aminoglycosides.

3.  The composition of claim 1 or 2, wherein the silver ions are present in an amount of 0.05 ppm to 50 ppm by weight of the dispersion, such as 0.05 ppm to 5 ppm by weight of the colloidal dispersion.

4.  The composition of any one of claims 1 to 3, wherein the silver ions have been adsorbed in an amount of 0.0005 to more than 5 silver ions per $nm^2$, such as from about 0.01 per $nm^2$ to more than 5 per $nm^2$, preferably wherein the concentration is 0.20 to 2.0 per $nm^2$, more preferably 0.50 to 1.50 $nm^2$, and even more preferably 0.70 - 1.25 ions per $nm^2$.

5.  A kit of parts comprising:

    a) a pharmaceutical formulation including an antibiotic agent, optionally in admixture with a pharmaceutically acceptable excipient; and
    b) a colloidal dispersion comprising particles of silica having a particle size from 3 nm to 25 nm to which particles silver ions have been adsorbed,
    wherein the antibiotic agent is selected from the group consisting of aminoglycosides, beta-lactams, steroid antibiotics, spectinomycin, chloramphenicol and rifampicin.

6.  The kit of claim 5, wherein the antibiotic agent is an antibiotic of the group aminoglycosides.

7.  The kit of claim 5 or 6, wherein the silver ions are present in an amount of 0.05 ppm to 50 ppm by weight of the dispersion, such as 0.05 ppm to 5 ppm by weight of the colloidal dispersion.

8.  The kit of any one of claims 5 to 7, wherein the silver ions have been adsorbed in an amount of 0.0005 to more than 5 silver ions per $nm^2$, such as from about 0.01 per $nm^2$ to more than 5 per $nm^2$, preferably wherein the concentration is 0.20 to 2.0 per $nm^2$, more preferably 0.50 to 1.50 $nm^2$, and even more preferably 0.70 - 1.25 ions per $nm^2$.

9.  A colloidal dispersion comprising particles of silica having a particle size from 3 nm to 25 nm to which particles silver ions have been adsorbed, for use in a therapeutic method for the treatment of a bacterial infection, by administration of said colloidal dispersion in combination with an antibiotic agent, wherein the antibiotic agent is selected from the group consisting of aminoglycosides, beta-lactams, steroid antibiotics, spectinomycin, chloramphenicol and rifampicin.

10. The colloidal dispersion for use of claim 9, wherein the antibiotic agent is an antibiotic of the group aminoglycosides.

11. The colloidal dispersion for use of claim 9 or 10, wherein said bacterial infection is an infection caused by gram-positive or gram-negative bacteria.

12. The colloidal dispersion for use of claim 11, wherein the bacterial infection is an infection by *Staphylococcous aureus, Pseudomonas aeruginosa, Salmonella enterica, Escherichia coli,* or *Acinetobacter baumannii.*

13. The colloidal dispersion for use of claim 11, wherein said antibiotic agent is an aminoglycoside, such as Gentamycin or Tobramycin, and the bacterial infection is an infection by *Pseudomonas aeruginosa* or *E.coli.*

14. The colloidal dispersion for use of claim 11 or 12, wherein said antibiotic agent is an aminoglycoside, and the bacterial infection is an infection by *Salmonella enterica.*

15. The colloidal dispersion for use of claim 11 or 12, wherein said antibiotic agent is a steroid antibiotic, such as Mupirocin or Fusidic acid, and the bacterial infection is an infection by *Staphylococcous aureus.*

16. The colloidal dispersion for use of any one of claims 9 to 15, wherein said administration is oral, rectal, topical, subcutaneous, intramuscular or pulmonary, optionally wherein the colloidal dispersion and the antibiotic agent are administered in sequence or concurrently.

17. The colloidal dispersion for use of any one of claims 9 to 16, in a method for the treatment of a bacterial infection of the skin or of the mucosa.


**Patentansprüche**

1. Pharmazeutische Zusammensetzung, umfassend:

   a) ein antibiotisches Mittel und
   b) eine kolloidale Dispersion von Siliciumdioxidpartikeln mit einer Partikelgröße von 3 nm bis 25 nm, an die Silberionen adsorbiert worden sind,

   wobei das antibiotische Mittel ausgewählt ist aus der Gruppe, bestehend aus Aminoglykosiden, Beta-Lactamen, Steroidantibiotika, Spectinomycin, Chloramphenicol und Rifampicin.

2. Zusammensetzung nach Anspruch 1, wobei das antibiotische Mittel ein Antibiotikum aus der Gruppe der Aminoglykoside ist.

3. Zusammensetzung nach Anspruch 1 oder 2, wobei die Silberionen in einer Menge von 0,05 ppm bis 50 ppm bezogen auf das Gewicht der Dispersion, wie zum Beispiel 0,05 ppm bis 5 ppm bezogen auf das Gewicht der kolloidalen Dispersion, vorliegen.

4. Zusammensetzung nach einem der Ansprüche 1 bis 3, wobei die Silberionen in einer Menge von 0,0005 bis mehr als 5 Silberionen pro $nm^2$ adsorbiert worden sind, wie zum Beispiel von etwa 0,01 pro $nm^2$ bis mehr als 5 pro $nm^2$, wobei die Konzentration vorzugsweise 0,20 bis 2,0 pro $nm^2$, stärker bevorzugt 0,50 bis 1,50 $nm^2$ und noch stärker bevorzugt 0,70 - 1,25 Ionen pro $nm^2$ beträgt.

5. Kit aus Teilen, umfassend:

   a) eine pharmazeutische Formulierung, die ein Antibiotikum einschließt, gegebenenfalls in Beimischung mit einem pharmazeutisch akzeptablen Hilfsstoff; und
   b) eine kolloidale Dispersion, die Siliciumdioxidpartikel mit einer Partikelgröße von 3 nm bis 25 nm umfasst, an die Silberionen adsorbiert worden sind,

   wobei das antibiotische Mittel ausgewählt ist aus der Gruppe, bestehend aus Aminoglykosiden, Beta-Lactamen, Steroidantibiotika, Spectinomycin, Chloramphenicol und Rifampicin.

6. Kit nach Anspruch 5, wobei das antibiotische Mittel ein Antibiotikum aus der Gruppe der Aminoglykoside ist.

7. Kit nach Anspruch 5 oder 6, wobei die Silberionen in einer Menge von 0,05 ppm bis 50 ppm bezogen auf das Gewicht der Dispersion, wie zum Beispiel 0,05 ppm bis 5 ppm bezogen auf das Gewicht der kolloidalen Dispersion, vorliegen.

8. Kit nach einem der Ansprüche 5 bis 7, wobei die Silberionen in einer Menge von 0,0005 bis mehr als 5 Silberionen pro $nm^2$ adsorbiert worden sind, wie zum Beispiel von etwa 0,01 pro $nm^2$ bis mehr als 5 pro $nm^2$, wobei die Konzentration vorzugsweise 0,20 bis 2,0 pro $nm^2$, stärker bevorzugt 0,50 bis 1,50 $nm^2$ und noch stärker bevorzugt 0,70 - 1,25 Ionen

pro nm$^2$ beträgt.

9. Kolloidale Dispersion, umfassend Siliciumdioxidpartikel mit einer Partikelgröße von 3 nm bis 25 nm, an die Silberionen adsorbiert wurden, zur Verwendung in einem therapeutischen Verfahren zur Behandlung einer bakteriellen Infektion durch Verabreichung der kolloidalen Dispersion in Kombination mit einem antibiotischen Mittel, wobei das antibiotische Mittel ausgewählt ist aus der Gruppe ausgewählt, bestehend aus Aminoglykosiden, Beta-Lactamen, Steroidantibiotika, Spectinomycin, Chloramphenicol und Rifampicin.

10. Kolloidale Dispersion zur Verwendung nach Anspruch 9, wobei das antibiotische Mittel ein Antibiotikum aus der Gruppe der Aminoglykoside ist.

11. Kolloidale Dispersion zur Verwendung nach Anspruch 9 oder 10, wobei die bakterielle Infektion eine Infektion ist, die durch grampositive oder gramnegative Bakterien verursacht wird.

12. Kolloidale Dispersion zur Verwendung nach Anspruch 11, wobei die bakterielle Infektion eine Infektion durch *Staphylococcus aureus, Pseudomonas aeruginosa, Salmonella enterica, Escherichia coli* oder *Acinetobacter baumannii* ist.

13. Kolloidale Dispersion zur Verwendung nach Anspruch 11, wobei das antibiotische Mittel ein Aminoglykosid, wie zum Beispiel Gentamycin oder Tobramycin, ist und die bakterielle Infektion eine Infektion durch *Pseudomonas aeruginosa* oder *E. coli* ist.

14. Kolloidale Dispersion zur Verwendung nach Anspruch 11 oder 12, wobei das antibiotische Mittel ein Aminoglykosid ist und die bakterielle Infektion eine Infektion durch *Salmonella enterica* ist.

15. Kolloidale Dispersion zur Verwendung nach Anspruch 11 oder 12, wobei das antibiotische Mittel ein Steroidantibiotikum, wie zum Beispiel Mupirocin oder Fusidinsäure, ist und die bakterielle Infektion eine Infektion durch *Staphylococcus aureus* ist.

16. Kolloidale Dispersion zur Verwendung nach einem der Ansprüche 9 bis 15, wobei die Verabreichung oral, rektal, topisch, subkutan, intramuskulär oder pulmonal erfolgt, wobei die kolloidale Dispersion und das antibiotische Mittel gegebenenfalls nacheinander oder gleichzeitig verabreicht werden.

17. Kolloidale Dispersion zur Verwendung nach einem der Ansprüche 9 bis 16 in einem Verfahren zur Behandlung einer bakteriellen Infektion der Haut oder der Schleimhaut.

**Revendications**

1. Composition pharmaceutique comprenant :

    a) un agent antibiotique ; et,
    b) une dispersion colloïdale de particules de silice ayant une taille de particule de 3 nm à 25 nm, particules sur lesquelles des ions argent ont été adsorbés,

    dans laquelle l'agent antibiotique est choisi dans le groupe constitué par les aminoglycosides, les bêta-lactamines, les antibiotiques stéroïdes, la spectinomycine, le chloramphénicol et la rifampicine.

2. Composition selon la revendication 1, dans laquelle l'agent antibiotique est un antibiotique du groupe des aminoglycosides.

3. Composition selon la revendication 1 ou 2, dans laquelle les ions argent sont présents en une quantité de 0,05 ppm à 50 ppm en poids de la dispersion, par exemple de 0,05 ppm à 5 ppm en poids de la dispersion colloïdale.

4. Composition selon l'une quelconque des revendications 1 à 3, dans laquelle les ions argent ont été adsorbés en une quantité de 0,0005 à plus de 5 ions argent par nm$^2$, par exemple d'environ 0,01 par nm$^2$ à plus de 5 par nm$^2$, de préférence dans laquelle la concentration est de 0,20 à 2,0 par nm$^2$, plus préférablement de 0,50 à 1,50 nm$^2$, et encore plus préférablement de 0,70 à 1,25 ions par nm$^2$.

**5.** Kit de pièces comprenant :

a) une formulation pharmaceutique comprenant un agent antibiotique, éventuellement en mélange avec un excipient pharmaceutiquement acceptable ; et
b) une dispersion colloïdale comprenant des particules de silice ayant une taille de particule de 3 nm à 25 nm, particules sur lesquelles des ions argent ont été adsorbés,

dans laquelle l'agent antibiotique est choisi dans le groupe constitué par les aminoglycosides, les bêta-lactamines, les antibiotiques stéroïdes, la spectinomycine, le chloramphénicol et la rifampicine.

**6.** Kit selon la revendication 5, dans lequel l'agent antibiotique est un antibiotique du groupe des aminoglycosides.

**7.** Kit selon la revendication 5 ou 6, dans lequel les ions argent sont présents en une quantité de 0,05 ppm à 50 ppm en poids de la dispersion, par exemple de 0,05 ppm à 5 ppm en poids de la dispersion colloïdale.

**8.** Kit selon l'une quelconque des revendications 5 à 7, dans lequel les ions argent ont été adsorbés en une quantité de 0,0005 à plus de 5 ions argent par $nm^2$, par exemple d'environ 0,01 par $nm^2$ à plus de 5 par $nm^2$, de préférence dans laquelle la concentration est de 0,20 à 2,0 par $nm^2$, plus préférablement de 0,50 à 1,50 $nm^2$, et encore plus préférablement de 0,70 à 1,25 ions par $nm^2$.

**9.** Dispersion colloïdale comprenant des particules de silice ayant une taille de particule de 3 nm à 25 nm, particules sur lesquelles des ions argent ont été adsorbés, pour une utilisation dans une méthode thérapeutique destinée au traitement d'une infection bactérienne, par administration de ladite dispersion colloïdale en association avec un agent antibiotique, dans laquelle l'agent antibiotique est choisi dans le groupe constitué par les aminoglycosides, les bêta-lactamines, les antibiotiques stéroïdes, la spectinomycine, le chloramphénicol et la rifampicine.

**10.** Dispersion colloïdale pour une utilisation selon la revendication 9, dans laquelle l'agent antibiotique est un antibiotique du groupe des aminoglycosides.

**11.** Dispersion colloïdale pour une utilisation selon la revendication 9 ou 10, dans laquelle ladite infection bactérienne est une infection provoquée par des bactéries à gram-positif ou à gram-négatif.

**12.** Dispersion colloïdale pour une utilisation selon la revendication 11, dans laquelle l'infection bactérienne est une infection par *Staphylococcus aureus, Pseudomonas aeruginosa, Salmonella enterica, Escherichia coli ou Acinetobacter baumannii.*

**13.** Dispersion colloïdale pour une utilisation selon la revendication 11, dans laquelle ledit agent antibiotique est un aminoglycoside, tel que la gentamicine ou la tobramycine, et l'infection bactérienne est une infection par *Pseudomonas aeruginosa* ou *E. coli.*

**14.** Dispersion colloïdale pour une utilisation selon la revendication 11 ou 12, dans laquelle ledit agent antibiotique est un aminoglycoside, et l'infection bactérienne est une infection par *Salmonella enterica.*

**15.** Dispersion colloïdale pour une utilisation selon la revendication 11 ou 12, dans laquelle ledit agent antibiotique est un antibiotique stéroïde, tel que la mupirocine ou l'acide fusidique, et l'infection bactérienne est une infection par *Staphylococcus aureus.*

**16.** Dispersion colloïdale pour une utilisation selon l'une quelconque des revendications 9 à 15, dans laquelle ladite administration est orale, rectale, topique, sous-cutanée, intramusculaire ou pulmonaire, éventuellement dans laquelle la dispersion colloïdale et l'agent antibiotique sont administrés en séquence ou simultanément.

**17.** Dispersion colloïdale pour une utilisation selon l'une quelconque des revendications 9 à 16, dans une méthode destinée au traitement d'une infection bactérienne de la peau ou des muqueuses.

A

B

Figure 1

Figure 2

Figure 2

Figure 3

Figure 4

Figure 5

Figure 6

## Fusidic acid (FUS)

AgSol=12.5 ppm (1/4xMIC)
FUS=0.25 μg/ml (2xMIC)

A)

## Mupirocin (MUP)

AgSol=12.5 ppm (1/4xMIC)
MUP=0.25 μg/ml (2xMIC)

B)

Figure 7

Lower inoculum (>$10^6$ CFU/ml)

A)

Higher inoculum (>$10^8$ CFU/ml)

B)

Figure 8

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008079149 A1
- WO 2008024422 A2
- WO 2008147427 A2
- WO 2009036714 A1
- WO 2011037523 A
- CN 105998062

### Non-patent literature cited in the description

- **LLER**. *The Chemistry of Silica*, 1979, 407-409
- Remington's Pharmaceutical Sciences. Mack Publishing Company
- Global Salm-Surv - A global Salmonella surveillance and laboratory support project. World Health Organization, April 2002
- **DYKHUIZEN, DANIEL E**. Experimental Studies of Natural Selection in Bacteria. *Annual Review of Ecology and Systematics*, 1990, vol. 21, 373-398